# EUROPEAN PATENT SPECIFICATION

(11) **EP 2 164 013 B1**
(45) Date of publication and mention of the grant of the patent: **01.03.2017**
(21) Application number: 08015448.7
(22) Date of filing: 02.09.2008
(51) Int. Cl.: G06F 19/00

(54) **Method and apparatus for setting a basal rate profile for an insulin pump**
Verfahren und Vorrichtung zum Festlegen des Profils der Basalrate einer Insulinpumpe
Procédé et appareil pour définir un profil de taux de base pour une pompe à insuline

(43) Date of publication of application: 17.03.2010
(73) Proprietor: F. Hoffmann-La Roche AG, 4070 Basel (CH); Roche Diabetes Care GmbH, 68305 Mannheim (DE)
(72) Inventor: von Büren, Daniel, 68300 St Louis (FR); Marcin, Stefan, 3422 Kirchberg (CH); Cris, Catalin, 3184 Wünnewil (CH); Haueter, Ulrich, 3506 Grosshöchstetten (CH)
(74) Representative: Rentsch Partner AG

(56) References cited:
- EP-A- 1 983 456
- US-A1- 2004 055 611
- US-A1- 2005 272 640
- US-A1- 2007 112 298
- US-A1- 2008 036 773
- US-B1- 6 208 355
- US-B2- 6 810 290
- ANONYMOUS: 'children with DIABETES - Presentations' INTERNET ARTICLE, [Online] 09 June 2008, XP055256713 Retrieved from the Internet: <URL:https://web.archive.org/web/2008060916 5927/http://www.childrenwithdiabetes.com/pr esentations/> [retrieved on 2016-03-09]
- JOHN WALSH: "Pumping How Do I Start?", FOCUS ON PUMPING CONFERENCE, 5 September 2004 (2004-09-05), XP055256711, WASHINGTON D. C. Retrieved from the Internet: URL:http://www.childrenwithdiabetes.com/pr esentations/Pump-Begin-CWD-0904a.ppt [retrieved on 2016-03-09]

## Description

The invention relates to a method and an apparatus for setting a basal rate profile for an insulin pump according to the preambles of the independent claims. A basal rate profile defines basal rate delivery for a selected time interval. It has a pre-defined number of profile segments each defining the basal rate delivery for a subset of the selected time interval (so called time interval subset). The basal rate profile may also be defined by a steady mathematical function. In this case each profile segment corresponds to a specific value of the mathematical function. The time interval subsets then correspond to the time instances associated with the values of the mathematical function. Hence, with the expression "profile segment" also a single value of a basale rate profile can be meant and with the expression "time interval subset" or "subset of the selected time interval" can also be meant a single time instance of the selected time interval.

A typical basal rate profile for an insulin pump comprises basal rate values for 24 hours, i.e. for an entire day. In case of diabetes, the basal rate defines a low rate of continuous insulin supply/delivery needed in particular for controlling cellular glucose and amino acid uptake. The basal rate is typically given in the unit IU/h (international unit per hour). The expression "basal rate profile" according to the present invention is also used for a part/time segment of another basal rate profile, e.g. of a 24-hour basal rate profile, that is stored in an insulin pump.

Using existing methods for programming and changing of basal rates often requires the manipulation of all profile segments of a stored basal rate profile. If the selected time interval is 24 hours and the subset of the selected time interval is 1 hour, then there are 24 profile segments. If the subset of the selected time interval is 30 minutes, then there are 48 profile segments. Having to manipulate all profile segments thus might lead to high programming effort that is often reduced by defining less profile segments which, however, might lead to simpler, blockier basal rate profiles and decreased preciseness of medical treatment. Furthermore, often the start basal rate and the end basal rate of a 24-hour basal rate profile are not adjusted such, that an immediate repetition of the same basal rate profile for example at midnight might entail an unintentional jump in the basal rate delivery.

Patent document US-B2-6 810 290 discloses a method for setting basal rates for an implantable insulin pump, wherein the delivery rates need not to be entered for each subset of the selected interval of time but only for those subsets that represent a change in delivery rate compared to a previous subset. The selected time interval is 24 hours beginning at midnight and the subset of the selected time interval is 30 minutes. Hence, each set of basal rates consists of 48 rates that can start on any half-hour of the day.

With today's on the market available personal computer software "ACCU-CHEK Insulin Pump Configuration Software Pro" a 24-hour basal rate profile with 24 profile segments can be set by either inputting all 24 basal rate values required for the 24 profile segments into the personal computer via a keyboard typing the values, or by pressing scroll buttons with respect to the values for all profile segments, or by pressing a mouse button, moving the mouse (thereby moving a cursor) and then releasing the mouse button, thereby increasing or decreasing bars with a cursor, each bar representing a profile segment. The personal computer on which the software is installed is connected to an insulin pump.

Furthermore, a basal rate profile can be set by defining a number of supporting points for the basal rate profile via an input unit of an insulin pump, generating a continuous function in accordance with the supporting points by means of a calculation unit of the insulin pump and generating a time sequence of basal rates from the continuous function by the calculation unit (confer European patent application no. 07 007 991.8).

It is an object of the invention to provide a method for setting a basal rate profile which is user-friendly, requires little effort and is easy to implement. It is a further object of the invention to provide an apparatus for performing the method of the invention. It is a further object of the invention to provide a method and an apparatus by which setting/programming of an physiologically inappropriate basal rate profile can be avoided; i.e. the method and the apparatus of the invention are robust/safe with respect to potential physiologically inappropriate basal rate profiles.

In order to implement these and still further objects of the invention, which will become more readily apparent as the description proceeds, a method for setting a basal rate profile for an insulin pump is provided that comprises the following steps: providing a curve that represents the basal rate as a continuous function of time as an input for an input unit and generating a basal rate profile from the curve by a calculation unit by assigning curve values at selected time instances to the number of profile segments of the basal rate profile. In case of the basal rate profile being defined as mathematical function the curve values at selected time instances are assigned by the calculation unit to corresponding values of the mathematical function, preferably at the same time instances. The insulin pump in this case controls basal rate delivery according to the mathematical function. Preferably, the mathematical function is an approximation of the provided curve which represents the basal rate.

Further, an apparatus for setting a basal rate profile according to the method of the invention is provided, that comprises an input unit for inputting a curve representing the basal rate as a continuous function of time, and a calculation unit for generating the basal rate profile from the curve by assigning curve values at selected time instances to the number of profile segments.

The basal rate profile preferably defines basal rate delivery for a 24-hour day as selected time interval. The curve values, i.e. the amplitude values of the provided curve with respect to the abscissa, constitute the basal delivery profile and the entire basal delivery volume is given by the integral of the curve over the selected time interval, in particular over 24 hours, i.e. an entire day. The subset of the selected time interval for each profile segment may be e.g. 1 hour resulting in 24 profile segments. It may also be e.g. 30 minutes resulting in 48 profile segments or 2 hours resulting in 12 profile segments. The choice of time interval subset for the profile segments may depend on the precision and/or the resolution of the employed insulin pump. The respective time interval subset may vary for each profile segment and in particular depend on the dynamics of the curve, i.e. its amplitude/absolute value variation.

The basal rate profile (and hence the curve) may also represent part of a 24-hour profile or another basal rate profile that is stored in the insulin pump and that is longer in time. The basal rate profile may also only comprise one profile segment, such that by setting a one-profile-segment basal rate profile only one profile segment of a longer basal rate profile, that is already stored in the insulin pump, is changed.

An insulin pump preferably performs basal delivery in a pulsed way with insulin pulses being delivered with a given time interval of e.g. 3 minutes. The number of profile segments may accordingly also be such that each profile segment defines the delivery with respect to one insulin pulse. For a time interval of 3 minutes, for example, this leads to 20 profile segments per hour.

The calculation unit preferably forms part of the insulin pump. The input unit may form part of the insulin pump. However, the input unit is preferably be given by a separate device, i.e. by a device separate and in particular remote from the insulin pump such as for example a dedicated remote controller, a personal digital assistant (PDA) or a personal computer (PC). The same applies to the calculation unit. Also the calculation unit is preferably given by a separate device and be integrated into e.g. a PDA or a PC. The input unit and the calculation unit may form part of the same separate device but do not have to.

The curve is preferably provided manually. Displaying the curve on a display area of the input device while or after it is provided is optional. The input device may be given by or comprise a touch screen with a display area. In this case the curve is provided by continuously moving an object such as a human finger or a stylus across the display area. The touch screen may form part of the insulin pump or it may form part of a separate device such as a PDA. The curve is preferentially drawn in one go without interruption from its beginning to its end, wherein it is basically irrelevant at which time instance the curve starts and how long in time the curve is. Removal of the object from the display area determines the curve end. Also the drawing direction (i.e. the direction in which the curve is drawn with respect to the time axis) is irrelevant and may be forward or backward in time.

The input device may also be given by or comprise a pointing device such as a mouse, a touchpad, a trackball or any other device that moves the cursor on the display area of a computer and allows the user to input continuous data (cf. http://en.wikipedia.org/wiki/Pointing_device). The curve is provided by continuously moving the pointing device and thereby moving the cursor on the display area e.g. of a PC that is used as input device. The curve is preferentially drawn in one go without interruption from its beginning to its end, wherein in case of the pointing device being a mouse the control/mouse button is pressed as long as the actual drawing takes place. It is again basically irrelevant at which time instance the curve starts and how long in time the curve is. The drawing direction may be forward or backward in time.

Alternatively, the input unit can also be given by or comprise a scanner or scanning device and the curve can be provided as a drawing that is then scanned by the scanner. The curve may be permanently drawn e.g. with a pen on a sheet of paper which is then placed in the scanner for scanning.

Each profile segment is preferably assigned that curve value whose time instance lies in the middle of the time interval subset of the respective profile segment. That is, for the duration of the time interval subset the curve value assigned to the profile segment corresponding to the time interval subset is kept constant (i.e. "frozen"). With the transition to the consecutive profile segment a different curve value may apply. Of course also another curve value, for example the curve value whose time instance corresponds to the starting time or the end time of the time interval subset of the respective profile segment, may be assigned to the respective profile segment. Also the average of the curve section whose time interval corresponds to the time interval subset of the respective profile segment may be assigned to the respective profile segment. Furthermore, the curve values that are assigned to the profile segments are preferably rounded, in particular according to a resolution (e.g. 0.1 IU) that is convenient for the design/mechanics of the employed insulin pump. The amplitude/height of a profile segment defines the basal rate delivery/discharge during its time interval subset.

Preferentially the provided curve is smoothed by filtering. For this the input unit and/or the calculation unit can have a filter unit such as filter that performs moving averaging or another low pass filter.

The method according to the invention can be straightforward implemented, with today's handling of insulin pumps still being possible. As the curve that represents the basal rates is provided preferably manually as continuous function of time, the generation of non-physiological basal rate profiles is less likely, a physiological basal rate profile being in particular defined as a continuous basal rate profile.

According to a preferred embodiment of the invention a monitoring unit is provided that may form part of the insulin pump. The monitoring unit may also be separate from the insulin pump. The deviation between curve values which are assigned to adjacent profile segments is monitored by the monitoring unit for an excess of a pre-defined threshold. A warning is generated by the monitoring unit if excess of the pre-defined threshold is detected. The first profile segment in time and the last profile segment in time are also considered as adjacent profile segments, in particular if basal rate delivery according to the basal rate profile shall be repeated in time. The warning can be in the form of a specific marking of a display of the profile segments on a display area of the input unit, i.e. a specific colouring/hatching of the concerned profile segments. If the pre-defined threshold is exceeded then the deviation between the curve values assigned to adjacent profile segments is physiologically deemed to be too large. The pre-defined threshold is preferably defined by the user. It can also be fixedly set by the manufacturer of the insulin pump. The type of the marking, in particular its intensity, distinctness and/or colour, preferably depends on the amount of deviation between curve values assigned to adjacent profile segments in case of an excess of the pre-defined threshold. The warning/marking may have the form of a sliding indication, wherein a colour, a gray shade or the like of the profile segments can be modified in a substantially continuous way (e.g. continuously from the colour white to the colour black) in dependence on the deviation or the amount of the deviation, respectively.

Besides the pre-defined threshold for the deviation between adjacent profile segments additional or alternative approaches may be used. In particular a threshold for the slope of the profile or parts of it rather than for the absolute deviation, which is especially useful if the length of the respective subsets (time intervals) of the profile segments is not constant), or an average deviation over a number of consecutive profile segments may be used. Futhermore, the value of the threshold may be different depending on whether heights/associated curve values are increasing or decreasing from one profile segment to the next profile segment.

Preferentially, an automatic correction of the threshold-exceeding adjacent profile segments is performed by the monitoring unit or the calculation unit, such that at least the amplitude value of one of the adjacent profile segments is drawn closer with regard to the height of the other profile segment. Alternatively, a proposal for corrected amplitude values/heights of the adjacent profile segments can be calculated and presented to a user for approval.

The generated basal rate profile with its profile segments is preferably presented together with the provided curve to a user on a display area of the input device. For presentation purposes means may be given for scrolling the base rate profile (and the curve if applicable) with respect to the time axis. A reference profile such as a profile according to Renner, Teupe or similar may be depicted on the display area to aid the user with the provision of the curve representing the basal rate. The reference profile may serve as exemplary curve. Additionally or alternatively to the reference profile other information deemed useful may be depicted, In particular, a curve of past blood glucose values of a patient/user and/or a curve of deviations of blood glucose values from a reference blood glucose value profile may be shown, as this may represents valuable for providing, in particular drawing, the basal rate curve, i.e. the curve representing the basal rate.

The generated basal rate profile may be further manipulated, e.g. for generation of a set of basal rate profiles. For manipulation purposes the basal rate profile may be multiplied by a specific factor and/or a specific constant may be added or subtracted from the basal rate profile. Another interesting manipulation is the shifting of the boarder(s) between adjacent profile segments in time domain. For generation of a second basal rate profile, a basal rate profile generated by the method of the invention may be copied and simultaneously scaled and/or shifted in one processing step (for example: already generated basal rate profile A is multiplied by the factor 1.2 to generate basal rate profile B). Furthermore, the dynamics of a generated base rate profile may be changed by scaling it with a specific scaling factor and/or shifted in time domain while keeping its mean value constant if applicable. In particular the manipulation can be performed by subtracting the mean value of the provided curve, scaling the intermediate curve re-suiting from the subtraction (which has a mean value of zero) with a specific factor, and adding to this (further) intermediate curve resulting from the scaling the mean value again. Thereby physiologically caused delays of the insulin taking effect may be advantageously compensated. Additionally, fixed boluses for the insulin may be set which are taken into account when generating the basal rate profile according to the method of the invention.

Further advantageous features and applications of the invention can be found in the dependent claims as well as in the following description of the drawings illustrating the invention. In the drawings like reference signs designate the same or similar parts throughout the several figures of which:
Fig. 1 shows a flowchart of the method according to the invention and
Fig. 2 shows a display area with a graph illustrating a provided curve representing basal rates and a basal rate profile generated by the method of the invention.

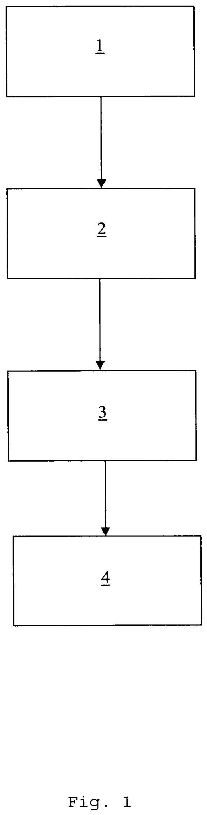

Figure 1 shows a flowchart of the method of the invention. In step 1 a curve representing a basal rate is provided as continuous function as an input for an input unit, for example by scanning a drawn curve, or by drawing a curve by hand on a display area of a touch screen or by means of a mouse on a display area of a personal computer, respectively. In step 2 this curve is optionally smoothed by filtering. In step 3 each profile segment of the basal rate profile to be generated is assigned a value of the curve, thereby generating the basal rate profile. The time instance of the respective curve value corresponds preferably to the middle time instance of the time interval subset of the respective profile segment. The deviation between curve values assigned to adjacent profile segments is monitored and the curve values are preferentially corrected if the deviation exceeds a pre-defined threshold. In optional step 4 the generated basal rate profile can be further manipulated e.g. by scaling in the time domain, shifting in the time domain, multiplication with a specific factor, adding/subtraction of a specific constant, while for example the basal delivery volume or/and its mean value kept constant if applicable. In particular the manipulation can be performed by subtracting the mean value of the provided curve, scaling the intermediate curve resulting from the subtraction (which has a mean value of zero) with a specific factor, and adding to this (further) intermediate curve resulting from the scaling the mean value again.

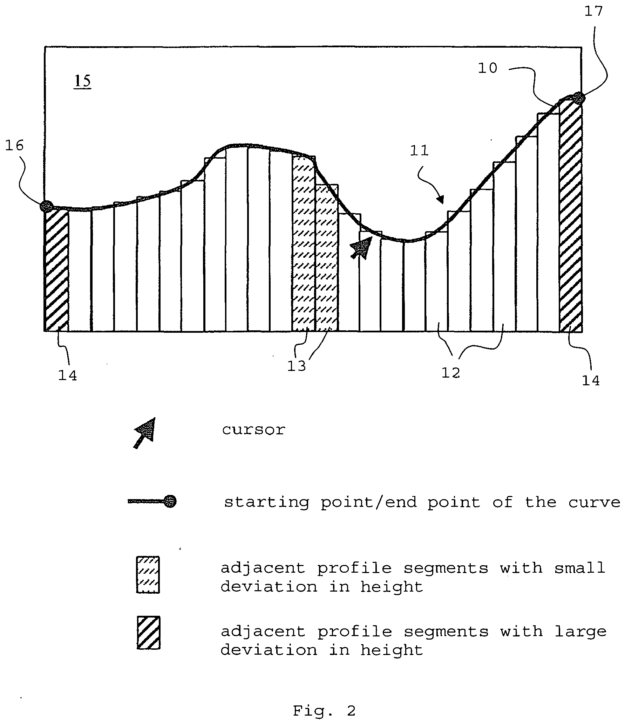

Figure 2 shows a display area 15 with graph illustrating a provided curve 10 representing basal rates and a basal rate profile 11 generated by the method of the invention. The basal rate profile 11 consists of 24 profile segments 12, 13, 14 and defines basal rate delivery exemplarily for 24 hours. Each profile segment 12, 13, 14 defines the basal rate delivery exemplarily for 1 hour. On the abscissa is given the time in hours. On the ordinate is given the basal rate in IU/h. The profile segments may have varying time durations, i.e. may be associated with different subsets of the selected overall time interval.

By moving the cursor 14 with a mouse (with its mouse button being pressed or, alternatively after first selecting a clearly marked so called action button or the like) or by moving a finger on a touch screen the curve 10 can be drawn on the display area 15. The starting time/starting point 16 of the curve is 10 defined by the first pressing of the mouse button or the first contact of the finger on the touch screen, respectively. The end time/end point 17 of the curve 10 is defined by releasing the mouse button or by removal of the finger from the touch screen, respectively. Adjacent profile segments 12 and 13 have a small deviation in height with does not exceed the pre-defined threshold and which therefore results in physiological basal rate delivery. The profile segments 14, which are also considered as adjacent profile segments as they are the first profile segment and the last profile segment in time and the generated basal rate profile is aimed for repetition without interruption, have a large deviation in height that exceeds the pre-defined threshold and is considered to be non-physiological. The heights of the profile segments 14 are therefore preferably corrected such that their deviation does not exceed the pre-defined threshold.

It is to be understood that while certain embodiments of the present invention have been illustrated and described herein, it is not to be limited to the specific embodiments described and shown.

## Claims

1. A method for setting a basal rate profile for an insulin pump, the basal rate profile (11) defining basal rate delivery for a selected time interval and having a pre-defined number of profile segments (12, 13, 14) each defining the basal rate delivery for a subset of the selected time interval, said method **characterized by** the following steps:
- providing a curve (10) by drawing, as a continuous function of time as an input for an input unit, the curve representing the basal rate, and
- generating the basal rate profile (11), that defines the basal rate delivery, from the curve (10) by a calculation unit by assigning curve values at selected time instances to the number of profile segments (12, 13, 14).

2. The method according to claim 1, wherein the input unit comprises a touch screen with a display area and the curve (10) is provided by continuously moving an object, in particular a finger, across the display area.

3. The method according to claim 1, wherein the input unit comprises a pointing device, in particular a mouse, for inputting continuous data and that the curve (10) is provided by continuously moving the pointing device.

4. The method according to claim 1, wherein the input unit comprises a scanner and the curve (10) is provided as a drawing scanned by the input unit.

5. The method according to one of the preceding claims, wherein to each profile segment (12, 13, 14) is assigned that curve value whose time instance lies in the middle of the time interval subset of the particular profile segment (12, 13, 14).

6. The method according to one the preceding claims, wherein the curve values that are assigned to the profile segments (12, 13, 14) are rounded according to a pre-defined resolution.

7. The method according to one of the preceding claims, wherein a monitoring unit is provided and the deviation between curve values assigned to adjacent profile segments (13, 14) is monitored by the monitoring unit for an excess of a pre-defined threshold, wherein a warning is generated by the monitoring unit if excess of the pre-defined threshold is detected.

8. The method according to claim 7, wherein the type of the warning, in particular the intensity and/or distinctness of the warning, depends on the amount of deviation.

9. The method according to one of the preceding claims, wherein a reference profile is depicted on a display area of the input unit.

10. The method according to one of the preceding claims, wherein the generated basal rate profile (11) is scaled with a specific scaling factor while its mean value is kept constant.

11. The method according to one of the preceding claims, wherein the provided curve (10) is smoothed by filtering.

12. An apparatus for setting a basal rate profile according to the method of one of the preceding claims, **characterized by** an input unit for inputting a curve (10) by drawing, the curve (10) representing the basal rate as a continuous function of time, and a calculation unit for generating the basal rate profile from the curve (11) by assigning curve values at selected time instances to the number of profile segments (12, 13, 14).

13. The apparatus according to claim 12, wherein the input unit comprises a touch screen with a display.

14. The apparatus according to claim 12, wherein the input unit comprises a pointing device, in particular a mouse, for in putting continuous data.

15. The apparatus according to claim 12, wherein the input unit comprises a scanner.

16. The apparatus according to one of the claims 12 to 15, wherein a monitoring unit is provided for monitoring the deviation between curve values assigned to adjacent profile segments (13, 14) for an excess of a pre-defined threshold and for generating a warning if excess of the pre-defined threshold is detected.

## Patentansprüche

1. Verfahren zum Einstellen eines Basalratenprofils für eine Insulinpumpe, wobei das Basalratenprofil (11) die Basalzuführung für ein ausgewähltes Zeitintervall definiert und eine vorbestimmte Anzahl von Profilsegmenten (12, 13, 14) aufweist, die jeweils die Basalzuführung für eine Untergruppe des ausgewählten Zeitintervalls definieren, wobei das Verfahren durch die folgenden Schritte gekennzeichnet ist:
- Bereitstellen einer Kurve (10) durch Zeichnen als eine kontinuierliche Funktion der Zeit, als eine Eingabe für eine Eingabeeinheit, wobei die Kurve die Basalrate repräsentiert, und
- Generieren des Basalratenprofils (11), das die Basalzuführung definiert, von der Kurve (10) durch eine Recheneinheit, indem Kurvenwerte zu ausgewählten Zeitpunkten der Anzahl der Profilsegmente (12,13,14) zugeordnet werden.

2. Verfahren nach Anspruch 1, wobei die Eingabeeinheit einen Touchscreen mit einem Anzeigenbereich umfasst und die Kurve (10) dadurch bereitgestellt wird, dass ein Gegenstand, insbesondere ein Finger, durchgängig über den Anzeigenbereich bewegt wird.

3. Verfahren nach Anspruch 1, wobei die Eingabeeinheit eine Zeigevorrichtung, insbesondere eine Maus, für die Eingabe von durchgängig Daten umfasst und die Kurve (10) durch fortwährendes Bewegen der Zeigevorrichtung bereitgestellt wird.

4. Verfahren nach Anspruch 1, wobei die Eingabeeinheit einen Scanner umfasst und die Kurve (10) als eine Zeichnung bereitgestellt wird, die durch die Eingabeeinheit gescannt wird.

5. Verfahren nach einem der vorhergehenden Ansprüche, wobei jedem Profilsegment (12, 13, 14) der Kurvenwert zugeordnet ist, dessen Zeitpunkt in der Mitte der Zeitintervall-Untergruppe des bestimmten Profilsegments (12,13,14) liegt.

6. Verfahren nach einem der vorhergehenden Ansprüche, wobei die Kurvenwerte, die den Profilsegmenten (12, 13, 14) zugeordnet sind, entsprechend einer vorbestimmten Auflösung aufgerundet werden.

7. Verfahren nach einem der vorhergehenden Ansprüche, wobei eine Überwachungseinheit bereitgestellt wird und die Abweichung zwischen Kurvenwerten, die angrenzenden Profilsegmenten (13, 14) zugeordnet sind, durch die Überwachungseinheit auf eine Überschreitung eines vorbestimmten Schwellenwerts überwacht wird, wobei durch die Überwachungseinheit eine Warnung erzeugt wird, wenn eine Überschreitung des vorbestimmten Schwellenwerts erkannt wird.

8. Verfahren nach Anspruch 7, wobei die Art der Warnung, insbesondere die Intensität und/oder die Deutlichkeit der Warnung von dem Ausmaß der Abweichung abhängt.

9. Verfahren nach einem der vorhergehenden Ansprüche, wobei ein Referenzprofil in einem Anzeigenbereich auf der Eingabeeinheit abgebildet wird.

10. Verfahren nach einem der vorhergehenden Ansprüche, wobei das generierte Basalratenprofil (11) mit einem bestimmten Skalierungsfaktor skaliert wird, während sein Mittelwert konstant gehalten wird.

11. Verfahren nach einem der vorhergehenden Ansprüche, wobei die bereitgestellte Kurve (10) durch Filtern geglättet wird.

12. Vorrichtung zum Einstellen eines Basalratenprofils gemäß dem Verfahren nach einem der vorhergehenden Ansprüche, **gekennzeichnet durch** eine Eingabeeinheit zum Eingeben einer Kurve (10) **durch** Zeichnen, wobei die Kurve (10) die Basalrate als eine durchgängige Funktion der Zeit repräsentiert, und eine Recheneinheit zum Generieren des Basalratenprofils von der Kurve (11), indem der Anzahl vom Profilsegmenten (12, 13, 14) an bestimmten Zeitpunkten Kurvenwerte zugeordnet werden.

13. Vorrichtung nach Anspruch 12, wobei die Eingabeeinheit einen Touchscreen mit einer Anzeige umfasst.

14. Vorrichtung nach Anspruch 12, wobei die Eingabeeinheit eine Zeigevorrichtung, insbesondere eine Maus, für die Eingabe von kontinuierlichen Daten umfasst.

15. Vorrichtung nach Anspruch 12, wobei die Eingabeeinheit einen Scanner umfasst.

16. Vorrichtung nach einem der Ansprüche 12 bis 15, wobei eine Überwachungseinheit für die Überwachung der Abweichung zwischen den Kurvenwerten, die den angrenzenden Profilsegmenten (13, 14) zugeordnet sind, auf eine Überschreitung eines vorbestimmten Schwellenwerts und zum Erzeugen einer Warnung, wenn eine Überschreitung des vorbestimmten Schwellenwerts erkannt wird, bereitgestellt wird.

## Revendications

1. Procédé de réglage d'un profil de taux de base pour une pompe à insuline, le profil de vitesse base (11) définissant l'administration à taux de base pour l'intervalle de temps sélectionné et ayant un nombre prédéfini de segments de profil (12, 13, 14) chacun définissant l'administration à taux de base pour un sous-ensemble de l'intervalle de temps sélectionné, ledit procédé étant **caractérisé par** les étapes suivantes :
- l'utilisation d'une courbe (10) par dessin, sous forme de fonction continue du temps en tant qu'entrée pour une unité d'entrée, la courbe représentant le taux de base, et
- la création du profil de taux de base (11), qui définit l'administration à vitesse de base, à partir de la courbe (10) par une unité de calcul par attribution des valeurs de courbe à des instants sélectionnés au nombre de segments de profil (12, 13, 14).

2. Procédé selon la revendication 1, dans lequel l'unité d'entrée comprend un écran tactile avec une zone d'affichage et où la courbe (10) s'obtient par déplacement continu d'un objet, en particulier d'un doigt, à travers la zone d'affichage.

3. Procédé selon la revendication 1, dans lequel l'unité d'entrée comprend un dispositif de pointage, en particulier une souris, pour entrer des données continues, et où la courbe (10) s'obtient par déplacement continu du dispositif de pointage.

4. Procédé selon la revendication 1, dans lequel l'unité d'entrée comprend un scanner et où la courbe (10) s'obtient sous forme de dessin scanné par l'unité d'entrée.

5. Procédé selon l'une quelconque des revendications précédentes, dans lequel à chaque segment de profil (12, 13, 14) est attribuée la valeur de courbe dont l'instant se trouve au milieu du sous-ensemble d'intervalles de temps du segment de profil particulier (12, 13, 14).

6. Procédé selon l'une quelconque des revendications précédentes, dans lequel les valeurs de courbes qui sont attribuées aux segments de profil (12, 13, 14) sont arrondies selon une résolution prédéfinie.

7. Procédé selon l'une quelconque des revendications précédentes, dans lequel une unité de surveillance est prévue et où l'écart entre les valeurs de courbe attribuées aux segments de profil adjacents (13, 14) est suivi par l'unité de surveillance pour un excès d'un seuil prédéfini, un avertissement étant produit par l'unité de surveillance si un excès du seuil prédéfini est détecté.

8. Procédé selon la revendication 7, dans lequel le type de l'avertissement, en particulier l'intensité et/ou la distinction de l'avertissement, dépend de l'ampleur de l'écart.

9. Procédé selon l'une quelconque des revendications précédentes, dans lequel un profil de référence est représenté sur une zone d'affichage de l'unité d'entrée.

10. Procédé selon l'une quelconque des revendications précédentes, dans lequel le profil de taux de base produit (11) est mis à l'échelle avec un facteur d'échelle spécifique tandis que sa valeur moyenne est maintenue constante.

11. Procédé selon l'une quelconque des revendications précédentes, dans lequel la courbe fournie (10) est lissée par filtrage.

12. Appareil de réglage d'un profil de taux de base selon le procédé de l'une quelconque des revendications précédentes, **caractérisé par** une unité d'entrée servant à entrer une courbe (10) par dessin de la courbe (10) représentant le taux de base sous forme de fonction continue du temps, et une unité de calcul servant à créer le profil de vitesse de base à partir de la courbe (11) par attribution de valeurs de courbe à des instants sélectionnés au nombre de segments de profil (12, 13, 14).

13. Appareil selon la revendication 12, dans lequel l'unité d'entrée comprend un écran tactile avec un affichage.

14. Appareil selon la revendication 12, dans lequel l'unité d'entrée comprend un dispositif de pointage, en particulier une souris, pour entrer des données continues.

15. Appareil selon la revendication 12, dans lequel l'unité d'entrée comprend un scanner.

16. Appareil selon l'une quelconque des revendications 12 à 15, dans lequel une unité de surveillance sert à suivre l'écart entre les valeurs de courbe attribuées aux segments de profil adjacents (13,14) pour un excès d'un seuil prédéfini et à produire un avertissement si l'excès du seuil prédéfini est détecté.
